(19) Europäisches Patentamt
European Patent Office
Office européen des brevets

(11) Veröffentlichungsnummer: **0 188 999**
**B1**

# (12) EUROPÄISCHE PATENTSCHRIFT

(45) Veröffentlichungstag der Patentschrift: **31.05.89**

(51) Int. Cl.⁴: **C 07 D 233/61**, D 06 P 5/06, D 06 P 1/66, C 07 D 405/12 // (C07D405/12, 203:00, 233:00)

(21) Anmeldenummer: **85810498.7**

(22) Anmeldetag: **30.10.85**

## (54) Kationische Umsetzungsprodukte aus 1-Aminoalkylimidazolverbindungen und Epihalogenhydrinen.

(30) Priorität: **21.01.85 CH 260/85**

(43) Veröffentlichungstag der Anmeldung: **30.07.86 Patentblatt 86/31**

(45) Bekanntmachung des Hinweises auf die Patenterteilung: **31.05.89 Patentblatt 89/22**

(84) Benannte Vertragsstaaten: **BE CH DE FR GB IT LI**

(56) Entgegenhaltungen:
**DD-A-136 269**
**DD-A-210 062**
**GB-A-2 094 298**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Töpfl, Rosemarie, Dorneckstrasse 68, CH- 4143 Dornach (CH)**

EP 0 188 999 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft 1-Aminoalkylimidazoliumverbindungen. Verfahren zu ihrer Herstellung und ihre Verwendung zur Verbesserung der Farbausbeute und der Nassechtheiten von mit anionischen Farbstoffen auf Cellulosefasermaterial erzeugten Färbungen oder Drucken.

Aus der GB-A-2 094 298 sind faserreaktive Imidazoliumverbindungen bekannt, in denen die Ringstickstoffatome durch eine Chlorhydrin oder Glycidylgruppe substituiert sind.

Gegenstand vorliegender Erfindung sind wasserlösliche 1-Aminoalkyl-Imidazolium-Verbindungen der Formel

(1) 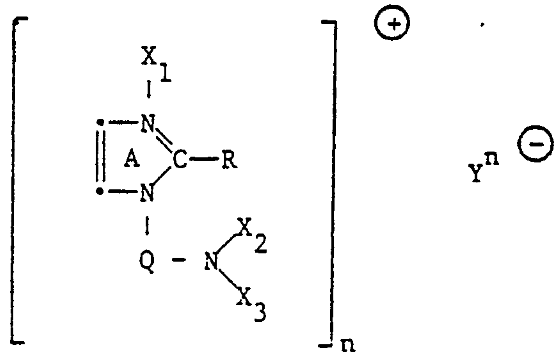

worin
in der

$X_1$ und $X_2$, unabhängig voneinander die Gruppe

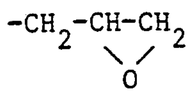

oder vorzugsweise
$-CH_2-CH-CH_2-Hal$,
|
OH

$X_3$ Wasserstoff, Niederalkyl,

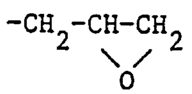

oder
$-CH_2-CH-CH_2-Hal$,
|
OH

Hal ein Halogenatom,
Q Alkylen mit 2 bis 4 Kohlenstoffatomen,
R Wasserstoff, Phenyl oder einen aliphatischen Rest,
n 1 oder 2 und
$Y^{\ominus}$ ein Anion einer starken anorganischen oder organischen Säure bedeuten und

der Imidazolring A unsubstituiert oder durch unsubstituiertes oder durch Halogen, Hydroxyl oder Cyano substituiertes Niederalkyl substituiert ist.

Halogen in Verbindung mit sämtlichen vor- und nachstehenden Substituenten bedeutet beispielsweise Brom, Fluor, Jod oder vorzugsweise Chlor.

Die faserreaktiven Gruppen $X_1$, $X_2$ und $X_3$ sind vorzugsweise identisch und sind in erster Linie

$-CH_2-CH-CH_2-Hal_1$ (Halogenhydringruppen),
|
OH

worin $Hal_1$ Brom oder vorzugsweise Chlor ist.

Vorzugsweise enthält die Imidazolverbindung zwei faserreaktive Gruppierungen, nämlich $X_1$ und $X_2$.

$X_3$ ist vor allem Wasserstoff.

Bei der Alkylengruppe Q handelt es sich beispielsweise um die

$-CH_2-CH_2-$, $-CH_2-CH_2-CH_2$,

$-CH_2-CH(CH_3)-$,

$-CH(CH_3)-CH_2-CH_2-$,

$-CH_2-CH(CH_3)-CH_2-$ oder $-CH_2-CH_2-CH_2-CH_2-$Gruppe. Vorzugsweise ist Q die Propylrngruppe $-CH_2-CH_2-CH_2-$.

Als aliphatischer Rest kann R gesättigt oder ungesättigt, geradkettig oder verzweigt sein und bis zu 23 Kohlenstoffatome aufweisen. Vorteilhafterweise ist R ein Alkylrest mit 1 bis 23 Kohlenstoffatomen.

Beispiele für solche Alkylreste sind Methyl, Ethyl, n-Propyl, Isopropyl, n-Butyl, Isobutyl n-Amyl, Isoamyl, Hexyl, Heptyl, Octyl, Isooctyl, Nonyl, Isononyl, Undecyl, Dodecyl, Tridecyl, Pentadecyl, Heptadecyl, Nonadecyl, Heneicosyl oder Tricosyl.

Vorzugsweise bedeutet R Wasserstoff, Niederalkyl, Undecyl oder Heptadecyl. Niederalkyl kann durch Halogen, Cyano oder Hydroxy substituiert sein.

Niederalkyl stellt in der Regel solche Gruppen oder Gruppenbestandteile dar, die 1 bis 5, insbesondere 1 bis 3 Kohlenstoffatome aufweisen, wie z. B. Methyl, Ethyl, n-Propyl, Isopropyl, n-Butyl, Isobutyl, tert. Butyl, n-Amyl, Isoamyl oder tert. Amyl.

Sind allfällige Niederalkylreste substituiert, so handelt es sich vor allem um Halogenalkyl, Cyanoalkyl oder Hydroxyalkyl, die jeweils 2 bis 4 Kohlenstoffatome aufweisen, wie z. B. 2-Chlorethyl, 2-Cyanoethyl, 2-Hydroxyethyl oder 3-Hydroxypropyl.

Bevorzugte Substituenten des Ringes A sind Methyl und Ethyl.

Als Anionen $Y^\ominus$ kommen sowohl Anionen anorganischer Säuren, wie z. B. das Chlorid-, Bromid-, Fluorid-, Jodid-, Sulfat- oder Phosphat-Ion als auch Anionen organischer Säuren, z. B. aromatischer oder aliphatischer Sulfonsäuren, wie z. B. Benzolsulfonat-, p-Toluolsulfonat-, Chlorbenzolsulfonat-, Methan- oder Ethansulfonat-Ion, ferner die Anionen niederer Carbonsäuren, wie z. B. Acetat-, Propionat- oder Oxalation.

$Y^\ominus$ ist vor allem das Chlorid-, Bromid- oder Sulfation.

Praktisch wichtige kationische Verbindungen entsprechen der Formel

(2) 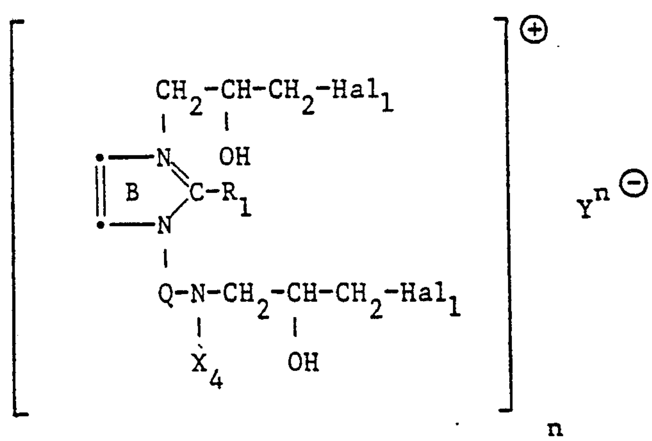

worin $Hal_1$, Q, $Y^\ominus$ und n die angegebene Bedeutung haben,

$R_1$ Wasserstoff, Phenyl oder Alkyl mit 1 bis 17 Kohlenstoffatomen und $X_4$ Wasserstoff oder $-CH_2-CH(OH)-CH_2-Hal_1$

bedeuten und der Ring B unsubstituiert oder durch Methyl oder Ethyl substituiert ist.

Von ganz besonderem Interesse sind Imidazolium-Verbindungen der Formel

(3) 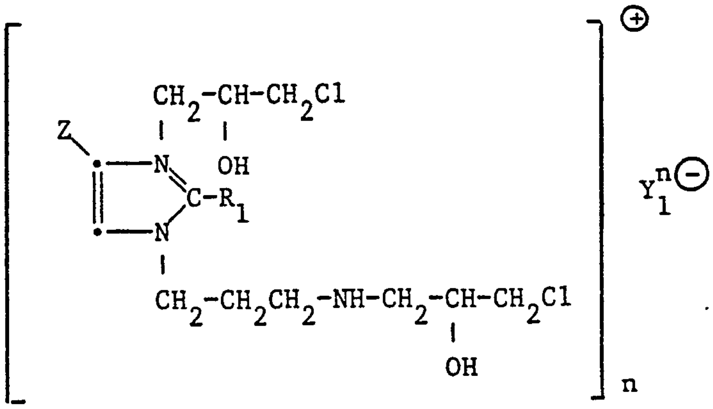

worin

$R_1$ Wasserstoff, Phenyl oder Alkyl mit 1 bis 17 Kohlenstoffatomen,
Z Wasserstoff oder Methyl,
n 1 oder 2 und
$Y_1^{n\ominus}$ das Chlorid- oder Sulfation bedeuten.

Unter den Imidazoliumverbindungen der Formeln (2) und (3) sind diejenigen, in denen $R_1$ Wasserstoff oder $C_1$-$C_{17}$-Alkyl bedeutet, bevorzugt.
Typische Vertreter der erfindungsgemässen Imidazolium-Verbindungen sind:

1-[3'-Chlor-2'-hydroxypropylamino-propyl]-3-(3'-chlor-2'-hydroxypropyl)-imidazoliumchlorid,
1-[Di-(3'-chlor-2'-hydroxypropyl)-amino-propyl]-3-(3'-chlor-2'-hydroxypropyl)-imidazoliumchlorid,
1-[3'-Chlor-2'-hydroxypropylamino-propyl]-3-(3'-chlor-2'-hydroxypropyl)-imidazoliumsulfat,
1-[Di-(3'-chlor-2'-hydroxypropyl)-amino-propyl]-3-(3'-chlor-2'-hydroxypropyl)-imidazoliumsulfat,
1-(3'-Chlor-2'-hydroxypropylamino-propyl)-3-(3'-chlor-2'-hydroxypropyl)-2-methyl-imidazoliumsulfat,
1-(3'-Chlor-2'-hydroxypropylamino-propyl)-3-(3'-chlor-2'-hydroxypropyl)-2-undecyl-imidazoliumsulfat,
1-(3'-Chlor-2'-hydroxypropylamino-propyl)-3-(3'-chlor-2'-hydroxypropyl)-2-heptadecyl-imidazoliumsulfat,
1-(3'-Chlor-2'-hydroxy propylamino-propyl)-3-(3'-chlor-2'-hydroxypropyl)-2-phenyl-imidazolium-sulfat.

Die Herstellung der Imidazoliumsalze erfolgt in an sich bekannter Weise. Vorzugsweise kann die Herstellung dadurch erfolgen, dass man 1 Mol einer entsprechenden 1-substituierten Imidazolverbindung oder vorzugsweise deren Säuresalzes z. B. mit Chlorwasserstoffsäure oder Schwefelsäure, mit 2 oder 3 Mol eines Epihalogenhydrins, wie z. B. Epibromhydrin, β-Methylepichlorhydrin oder vorzugsweise Epichlorhydrin umsetzt.

Die Umsetzungsbedingungen für die Herstellung der Imidazoliumsalze sind so zu wählen, dass weder infolge zu hoher pH-Werte des Reaktionsmediums noch infolge zu hoher Temperatur ein vorzeitiger Austausch beweglicher Substituenten eintritt. Man arbeitet daher bevorzugt in verdünntem, wässerigem Medium unter möglichst schonender Temperatur und pH-Verhältnissen zweckmässig bei einer Temperatur von 30 bis 95°C und einem pH-Wert von 5 bis 8 vorzugsweise 5,5 bis 7, wobei zur Erzielung des erwünschten pH-Wertes eine Halogenwasserstoffsäure, wie z. B. Chlorwasserstoffsäure oder Schwefelsäure hinzugefügt werden kann.

Als Imidazolkomponente für Herstellung der erfindungsgemässen Imidazoliumsalze kommen z. B. 1-Aminoethyl-imidazol, 1-Aminopropyl-imidazol, 1-Aminobutyl-imidazol, 1-Aminopropyl-2-methyl-imidazol, 1-Aminopropyl-2-isopropyl-imidazol, 1-Aminopropyl-2,4-dimethyl-imidazol, 1-Aminopropyl-4-methyl-imidazol, 1-Aminopropyl-2-phenyl-imidazol, 1-Aminopropyl-2-heptyl-imidazol, 1-Aminopropyl-2-undecyl-imidazol, 1-Aminopropyl-4-methyl-2-ethyl-imidazol, 1-Aminopropyl-4,5-dimethyl-2-undecylimidazol, 1-Aminopropyl-4-ethyl-2-undecyl-imidazol, 1-Aminopropyl-2-heptadecyl-imidazol und die entsprechenden Säuresalze in Betracht.

1-Aminopropyl-2-methylimidazol, 1-Aminopropyl-2-phenylimidazol, 1-Aminopropyl-2-heptadecyl-imidazol und vor allem 1-Aminopropylimidazol sind besonders bevorzugt.

Erfindungsgemässe Imidazoliumsalze eignen sich insbesondere zur Verbesserung der Farbausbeute und der Nassechtheiten von Färbungen oder Drucken, welche auf Cellulosefasermaterialien mit anionischen Farbstoffen erzeugt werden.

Die Behandlung des Cellulosefasermaterials mit der kationischen Imidazolverbindung erfolgt vorzugsweise kontinuierlich nach einem Foulardierverfahren. Dabei wird das Cellulosefasermaterial mit dem Fixiermittel imprägniert z. B. durch Klotzen, und dann einem Fixierprozess unterworfen. Diese Applikation kann vor dem Färben, während des Färbens oder nach dem Färben durchgeführt werden. Bevorzugt ist die Behandlung nach dem Färben. Die Nachbehandlung kann sowohl auf Färbungen als auch auf Farbdrucken durchgeführt werden.

Die Imprägnierstufe kann bei 20 bis 70°C, vorzugsweise jedoch bei Raumtemperatur vorgenommen werden.

Die Fixierstufe kann durch ein Dämpfverfahren, einen Thermosolierprozess, eine Mikrowellenbehandlung oder ein Warm- oder Kaltverweilverfahren durchgefürt werden.

Beim Dämpfverfahren werden die mit der Behandlungsflotte geklotzten Textilmaterialien einer Fixierbehandlung in einem Dämpfer mit gegebenenfalls überhitztem Dampf, zweckmässigerweise bei einer Temperatur von 98 bis 130°C, vorzugsweise 102 - 110°C unterzogen.

Die Fixierung durch den sogenannten Thermosolierprozess kann, nach einer oder ohne Zwischentrocknung, z. B. bei einer Temperatur von 100 bis 210°C erfolgen. Vorzugsweise erfolgt die Thermosolierung bei einer Temperatur von 120 bis 210°C, vorzugsweise 140 bis 180°C und nach einer Zwischentrocknung bei 80 bis 120°C der geklotzten oder bedruckten Ware. Je nach der Temperatur kann die Thermosolierung 20 Sekunden bis 5 Minuten, vorzugsweise 30 bis 180 Sekunden dauern.

Die Thermofixierung kann auch mittels Mikrowellen erfolgen. Hierbei wird die Ware nach Imprägnierung mit der Behandlungsflotte und Abquetschen des Feuchtigkeitsüberschusses, zweckmässigerweise aufgerollt, und in einer Kammer mittels Mikrowellen behandelt. Diese Mikrowellenbehandlung kann 2 bis 120 Minuten dauern. Vorzugsweise reichen 2 bis 15 Minuten aus. Als Mikrowellen bezeichnet man elektromagnetische Wellen (Radiowellen) im Frequenzbereich von 300 bis 100'000 MHz, vorzugsweise 1000 bis 30'000 MHz.

Beim Warmverweilverfahren lässt man die geklotzte oder bedruckte Ware in feuchtem Zustand z. B. 15 bis 120 Minuten lang, vorteilhafterweise bei Temperaturen von 85 bis 102°C verweilen. Hierbei kann die imprägnierte Ware durch eine Infrarot-Behandlung auf 85 bis 102°C vorgeheizt werden. Bevorzugt beträgt die Verweiltemperatur 90 bis 100°C.

Die Fixierstufe kann auch gemäss dem Kaltverweilverfahren durch Lagerung der geklotzten oder bedruckten und vorzugsweise aufgerollten Ware bei Raumtemperatur 15 bis 30°C z. B. während 3 bis 24 Stunden erfolgen. Gegebenenfalls kann auch bei leicht erhöhter Temperatur (30 bis 80°C) gelagert werden.

Die kontinuierliche Behandlung nach dem Färben wird bevorzugt durch Foulardieren des gefärbten oder bedruckten Fasermaterials mit dem anschliessenden Thermosolierprozess durchgeführt.

Die Behandlung der Textilmaterialien mit den Imidazoliumsalzen kann auch im Ausziehverfahren vor-, während oder nach dem Färben, insbesondere jedoch vor oder nach dem Färben, durchgeführt werden. Man kann in diesem Falle bei Temperaturen im Bereich von 20 bis 135°C, vorzugsweise 40 bis 100°C arbeiten. Das Flottenverhältnis kann innerhalb eines weiten Bereiches gewählt werden, z. B. 1 : 2,5 bis 1 : 100, vorzugsweise 1: 5 bis 1 : 40.

Die Behandlungsflotten enthalten die Imidazoliumsalze im Ausziehverfahren, vorzugsweise in einer Menge von 0,1 bis 25 Gew.-%, insbesondere 2 bis 15 Gew.-%, bezogen auf das Gewicht des Cellulosefasermaterials, oder bei Klotzflotten oder Druckpasten 1 bis 100 g/l, vorzugsweise 10 bis 50 g/l, wobei bei den Foulardierverfahren der Abquetscheffekt zweckmässig 60 bis 120 Gew.-% beträgt.

Bei den verwendeten Farbstoffen handelt es sich um die üblicherweise zum Färben von Cellulose-Textilmaterialien verwendbaren substantiven Farbstoffen oder vor allem Reaktivfarbstoffe.

Als Substantivfarbstoffe sind die üblichen Direktfarbstoffe geeignet, beispielsweise die in Colour Index, 3. Auflage (1971) Band 2 auf den Seiten 2005 - 2478 genannt "Direct Dyes".

Unter Reaktivfarbstoffen werden die üblichen Farbstoffe verstanden, welche mit der Cellulose eine chemische Bindung eingehen, z. B. die in Colour Index, in Band 3 (3. Auflage, 1971) auf den Seiten 3391 - 3560 und in Band 6 (revidierte 3. Auflage, 1975) auf den Seiten 6268 - 6345 aufgeführten "Reactive Dyes".

Bei gleichzeitiger Färbung und Behandlung mit den Imidazoliumsalzen richtet sich die Menge der Farbstoffe in der Regel nach der gewünschten Farbstärke und beträgt im kontinuierlichen Verfahren zweckmässig 0,1 bis 100 g pro Liter Flotte, vorzugsweise 5 bis 60 g/l Flotte. Im Ausziehverfahren beträgt die Farbstoffmenge vorteilhafterweise 0,1 bis 10 Gew.-%, vorzugsweise 1 bis 6 Gew.-%.

Ausser der kationischen Imidazoliumsalze enthalten die Flotten noch alkalisch reagierende Verbindungen, wie z. B. Natriumcarbonat, Natriumbicarbonat, Natriumhydroxid, Dinatriumphosphat, Trinatriumphosphat, Borax, wässeriges Ammoniak oder Alkalispender, wie z. B. Natriumtrichloracetat oder Natriumformiat. Die Einsatzmenge der Alkalien beträgt für Alkalimetallhydroxide vorzugsweise 3 bis 9 g/l Flotte und für Alkalimetallcarbonate vorzugsweise 8 - 25 g/l Flotte.

Der pH-Wert der Behandlungs- bzw. Färbeflotten beträgt in der Regel 8 bis 13,5, vorzugsweise 9,5 bis 13.

Gewünschtenfalls können die Flotten auch Harnstoff, Glycerin, Natriumformiat, Elektrolyte, wie z. B. Natriumchlorid oder Natriumsulfat, alkalibeständige Netzmittel, Homopolymerisate oder Mischpolymerisate des Acrylamids oder Methacrylamids oder Pfropfpolymerisate, wie sie in EP-A-111454 beschrieben sind, sowie auch Verdickungsmittel, wie z. B. Alginate, Stärkeether oder Johannisbrotkernmehlether enthalten.

Die erfindungsgemässen Imidazoliumsalze eignen sich für die Behandlung von Textilien die aus Cellulose bestehen oder diese enthalten.

Als Cellulosefasermaterial kommt solches aus regenerierter oder insbesondere natürlicher Cellulose in Betracht, wie z. B. Zellwolle, Viskoseseide, Celluloseacetat, Hanf, Leinen, Jute oder vorzugsweise Baumwolle, sowie Fasermischungen z. B. solche aus Polyamid/Baumwolle oder insbesondere aus Polyester/Baumwolle, wobei der Polyesteranteil gegebenenfalls vor- oder nachgefärbt werden kann.

Das Textilgut kann in beliebiger Form vorliegen, wie z. B. als Garn, Garnstrang, Gewebe, Gewirke, Filz, vorzugsweise in Form von textilen Flächengebilden wie Gewebe oder Maschenware, die ganz oder teilweise aus nätiver, regenerierter oder modifizierter Cellulose bestehen.

Man erhält unter Verwendung der erfindungsgemässen Imidazoliumsalze gleichmässige und farbkräftige Ausfärbungen, die sich gegenüber bekannten Verfahren durch eine verbesserte Farbausbeute auszeichnen.

Insbesondere werden Färbungen und Drucke auf Cellulosefasermaterial mit Reaktiv- als auch mit Substantivfarbstoffen erzeugt, welche eine erhebliche Verbesserung der Nassechtheiten zeigen. Zudem wird die Reissfestigkeitsabnahme der Färbungen durch den Einsatz der definitionsgemässen Imidazoliumsalze nicht beeinträchtigt. Je nach Einsatzmengen können diese Imidazoliumsalze gleichzeitig dem Cellulosefasermaterial Knitterfestigkeit und Krumpfechtheit anstelle von zusätzlichen entsprechenden Veredlungsmitteln verleihen.

In den folgenden Beispielen beziehen sich die Prozentansätze, wenn nichts anderes angegeben ist, auf das Gewicht. Die Mengen beziehen sich bei den Farbstoffen auf handelsübliche, d.h. coupierte Ware und bei den Hilfsmitteln auf Reinsubstanz. Die fünfstelligen Colour-Index Nummern (C.I.) beziehen sich auf die 3. Auflage des Colour-Index.

**Herstellungsbeispiele**

Beispiel 1

37,5 g 1-(3-Aminopropyl)-imidazol werden in 78 g Wasser und 15,3 g Schwefelsäure (96 %) gelöst worauf die Temperatur auf 60°C steigt. Hierauf lässt man auf diese Lösung 55,5 g Epichlorhydrin im Verlaufe von 60 Minuten zutropfen, wobei die Temperatur zwischen 60 und 66°C gehalten wird. Nach beendetem Zutropfen wird das Reaktionsgemisch noch 2 Stunden bei 65°C gerührt. Nach dieser Zeit beträgt der Epoxydgehalt: 0. Die erhaltene Lösung wird zur Trockene eingedampft. Man erhält 106 g 1-(3'-Chlor-2'-hydroxypropylaminopropyl)-3-(3'-chlor-2'-hydroxypropyl)-imidazoliumsulfat.

Beispiel 2

42,84 g 1-(3-Aminopropyl)-2-methyl-imidazol werden in 83 g Wasser und 15,3 g Schwefelsäure (96 %) gelöst, worauf die Temperatur auf 59°C steigt. Hierauf lässt man auf diese Lösung 55,5 g Epichlorhydrin im Verlaufe von 40 Minuten zutropfen, wobei die Temperatur zwischen 65 und 70°C gehalten wird. Nach beendetem Zutropfen wird das Reaktionsgemisch noch 2 1/2 Stunden bei 65°C gerührt. Nach dieser Zeit beträgt der Epoxydgehalt 0. Das Reaktionsgemisch wird zur Trockene eingedampft. Man erhält 113 g 1-(3'-Chlor-2'-hydroxypropylaminopropyl)-3-(3'-chlor-2'-hydroxypropyl)-2-methyl-imidazoliumsulfat.

Beispiel 3

66,9 g 1-(3-Aminopropyl)-2-phenylimidazol werden in 107 g Wasser und 15,3 g Schwefelsäure (96 %) gelöst, wobei die Temperatur auf 53°C ansteigt. Hierauf lässt man auf diese Lösung 55,5 g Epichlorhydrin im Verlaufe von 40 Minuten zufliessen, wobei die Temperatur zwischen 65 und 70°C gehalten wird. Nach beendetem Zufluss wird das Reaktionsgemisch noch 5 1/2 Stunden bei 68°C gerührt. Nach dieser Zeit beträgt der Epoxydgehalt 0. Das Reaktionsgemisch wird zur Trockene eingedampft. Man erhält 137 g 1-(3'-Chlor-2'-hydroxypropylaminopropyl)-3-(3'-chlor-2'-hydroxypropyl)-2-phenyl-imidazoliumsulfat.

Beispiel 4

38,9 g 1-(3-Aminopropyl)-2-heptadecylimidazol werden mit 145 g Wasser und 5,1 g Schwefelsäure (96 %) gemischt und auf 60°C erwärmt. Hierauf lässt man 18,5 g Epichlorhydrin im Verlaufe von 25 Minuten zutropfen. Nach beendetem Zufluss wird das Reaktionsgemisch 1 1/2 Stunden bei 78°C gerührt. Nach dieser Zeit beträgt der Epoxydgehalt 0. Man erhält 62 g 1-(3'-Chlor-2'-hydroxypropyl-aminopropyl)-3-(3'-chlor-2'-hydroxypropyl)-2-heptadecyl-imidazoliumsulfat.

Beispiel 5

25 g 1-(3-Aminopropyl)-imidazol werden in 70,3 g Wasser und 10,2 g Schwefelsäure (96 %) gelöst, worauf die Temperatur auf 59°C ansteigt. Dann lässt man 55,5 g Epichlorhydrin im Verlaufe von 30 Minuten zutropfen, wobei die Temperatur zwischen 65 und 70°C gehalten wird. Nach beendetem Zutropfen wird das Reaktionsgemisch noch 1 Stunde bei 70°C gehalten. Nach dieser Zeit beträgt der Epoxydgehalt 0. Das Reaktionsgemisch wird zur Trockene eingedampft. Man erhält 90 g 1-[Di-(3'-chlor-2'-hydroxypropyl-)amino-propyl-3-(3'chlor-2'hydroxypropyl)-imidazoliumsulfat.

**Anwendungsbeispiele**

Beispiel 1

In einem Kreuzspulfärbeapparat werden 500 g Baumwollgarn in 5 Liter Wasser eingenetzt, worauf die Flotte auf 95 bis 98°C erwärmt wird. Alsdann werden 20 g eines Farbstoffes der Formel

(11) 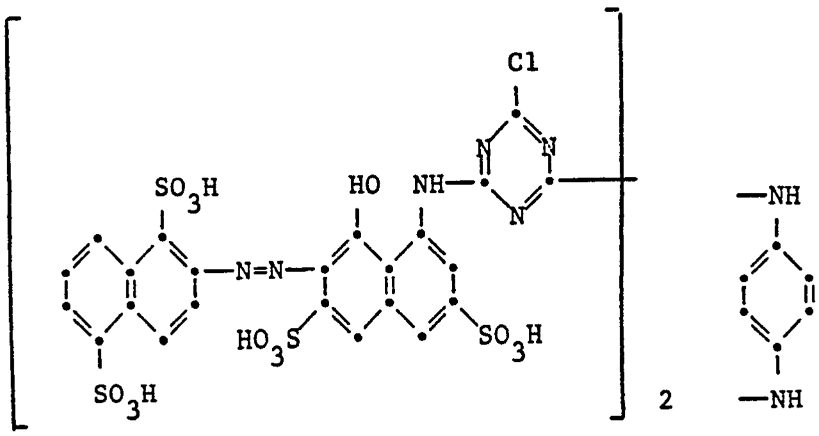

und 400 g Natriumsulfat zugegeben. Man kühlt auf 80°C ab und gibt 10 ml einer wässerigen 30 %-igen Natriumhydroxidlösung und 25 g Natriumcarbonat zu. Man kühlt weiter auf 40°C ab und fügt 60 ml der 30 %-igen Natriumhydroxidlösung und 50 g des gemäss dem Beispiel 1 hergestellten 1-(3'-Chlor-2'-hydroxypropylamino-propyl)-3-(3'-chlor-2-hydroxypropyl)-imidazoliumsulfates hinzu. Hiernach wird das Textilgut noch 60 Minuten bei 40°C behandelt und anschliessend gespült und getrocknet.

Man erhält eine gleichmässige und farbkräftige rote Färbung mit erhöhter Farbausbeute und hervorragenden Nassechtheiten. Die ISO-C2S-Wäsche ist deutlich verbessert.

Beispiel 2

In einem Kurzflotten-Jet werden 100 g Laumwollgewebe mit 800 g Wasser eingenetzt. Hierauf wird das Bad auf 40°C erwärmt und mit 3 g eines Direktfarbstoffes der Formel

(12) 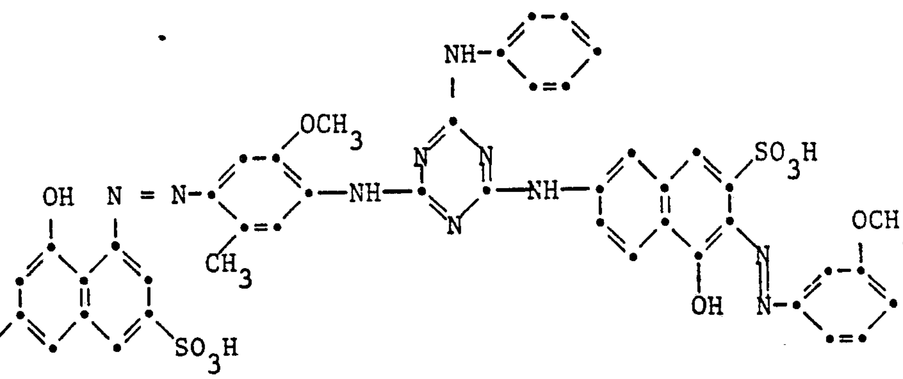

und 48 g Natriumsulfat versetzt. Nach 10 Minuten werden bei gleicher Temperatur 9,6 g einer wässrigen 30 % Natriumhydroxidlösung und 8 g des gemäss Beispiel 1 hergestellten 1-(3'-Chlor-2'-hydroxy-propylanino-propyl)-3-(3'-chlor-2'-hydroxypropyl)-imidazolium-sulfates zugegeben. Das Gewebe wird dann noch 60 Minuten bei 40°C behandelt und anschliessend gespült und getrocknet.

Man erhält eine gleichmässige und farbstarke rote Färbung mit einer 50 %-igen Ausbeutesteigerung. Die ISO-C2S-Wäsche zeigt die Note 4.

Beispiel 3

Ein Baumwollgewebe wird auf einem Foulard mit einer Flotte, die im Liter

60 g des Farbstoffes der Formel

(13) 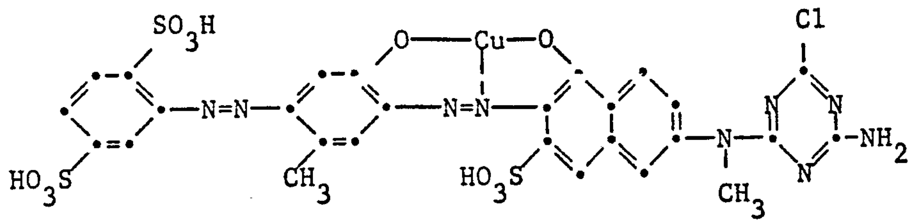

| | |
|---|---|
| 100 g | Harnstoff |
| 35 g | des gemäss Beispiel 1 hergestellten 1-(3'-Chlor-2'-hydroxypropyl-aminopropyl)-3-(3'-chlor-2'-hydroxypropyl)-imidazoliumsulfates |
| 20 g | Natriumcarbonat |
| 3 g | Natriumsalz der 3-Nitrobenzolsulfonsäure |

enthält, imprägniert. Die Flotteaufnahme beträgt 80 %. Hiernach wird das Gewebe aufgerollt und 18 Stunden bei Raumtemperatur gelagert. Alsdann wird das Gewebe heiss und kalt gewaschen und getrocknet.

Man erhält eine farbstarke und gleichmässig blaue Färbung, die eine 20 % Verbesserung der Farbausbeute aufweist. Nach Aufbewahrung in einer mit Wasserdampf gesättigten Atmosphäre während 3 Tagen bei 60°C verursacht die Färbung kein Anbluten des Begleitgewebes (Hydrolysentest vor der ISO-C2S-Wäsche). Die ISO-C2S-Wäsche ist auch nach dem Hydrolysentest 4.

Beispiel 4

Ein Baumwolltricot wird mit einer Flotte, welche im Liter

12 g des Farbstoffes der Formel

(14) 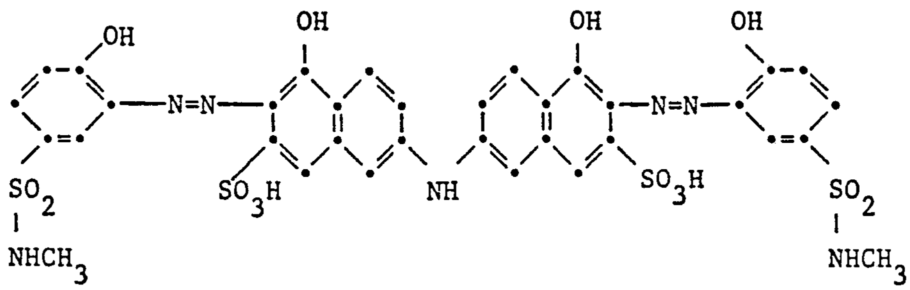

| | |
|---|---|
| 100 g | Harnstoff |
| 20 g | Natriumhydroxidlösung (30 %) |
| 26 g | des gemäss Beispiel 1 hergestellten 1-(3'-Chlor-2'-hydroxypropylaminopropyl-)-3-(3'-chlor-2'-hydroxypropyl)-imidazoliumsulfates |

enthält, foulardiert. Danach wird die Ware aufgerollt, luftdicht verpackt und während 20 Stunden bei Raumtemperatur gelagert. Anschliessend wird das Textilgut gespült und getrocknet. Man erhält eine farbstarke violette Färbung. Die Ausbeutesteigerung ist 100 %. Zudem sind die Nassechtheiten der Färbung ausgezeichnet. Das Begleitgewebe wird im ISO-C2S-Wäsche-Test und bei der Prüfung der Nassbügelechtheit praktisch nicht angefärbt.

Beispiel 5

Eine Färbung, die auf Baumwolltricot mit 6 % des Farbstoffes der Formel (II) erzeugt worden ist, wird mit einer Flotte, welche im Liter

| | |
|---|---|
| 26 g | des gemäss Beispiel 1 hergestellten 1-(3'-Chlor-2'-hydroxypropylamino-propyl)-3-(3'-chlor-2'-hydroxypropyl)-imidazoliumsulfates und |
| 15 ml | Natriumhydroxidlösung (30 %) |

enthält, foulardiert. Die Flottenaufnahme ist 85 %. Hiernach wird die Ware bei 90°C getrocknet und dann während 3 Minuten zwecks Fixierung bei 140°C behandelt.

Nach einem Hydrolysentest zeigt die Ware die Note 4 in der ISO-C2S-Wäsche.

Beispiel 6

Eine auf konventionelle Weise mit 5 % eines Farbstoffes der Formel

(15) 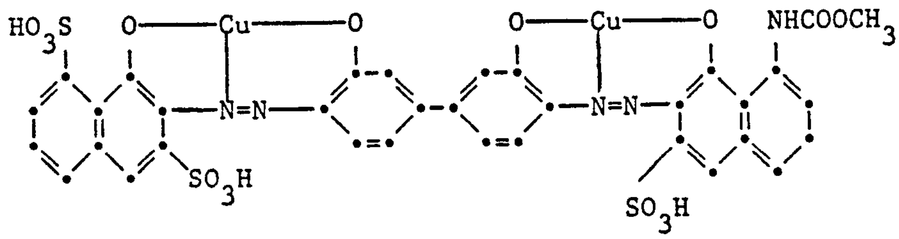

fertig gestellte Färbung wird wie in Beispiel 5 beschrieben nachbehandelt. Die deste für die ISO-CS 2-Wäsche und die Nassbügelechtheit zeigen (im Gegensatz zu einer konventionell nachfixierten Färbung) kein Anbluten des Begleitgewebes.

Beispiel 7

Ein Baumwollgewebe wird vor dem Färben mit einer Flotte foulardiert, die im Liter

| | |
|---|---|
| 26 g | des gemäss Beispiel 1 hergestellten 1-(3'-Chlor-2'-hydroxypropylaminopropyl)-3-(3'-chlor-2'-hydroxypropyl)-imidazoliumsulfates |
| 25 ml | Natriumhydroxidlösung (30 %) |

enthält. Hierauf wird die Ware bei 90° C getrocknet und 3 Minuten bei 140° C thermosoliert.

1 kg des vorbehandelten Gewebes wird in 30 Liter Wasser bei 25° C eingenetzt, worauf 30 g eines Farbstoffes der Formel

(16) 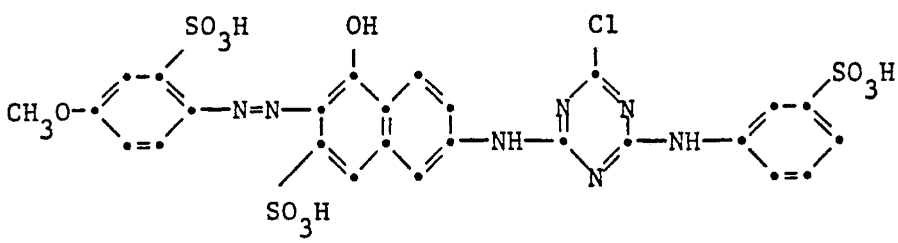

600 g Natriumcarbonat und 90 ml Natriumhydroxidlösung (30 %) zugegeben werden.

Die Färbeflotte wird im Verlaufe von 40 Minuten auf 80°C erwärmt und 60 Minuten bei dieser Temperatur gehalten. Anschliessend wird das gefärbte Gewebe heiss und kalt gespült und getrocknet.

Durch die Vorbehandlung der Ware mit dem Imidazoliumsalz wird die Farbstoffausbeute um 60 % verbessert. Die Färbung zeigt zudem hervorragende Nassechtheiten.

Beispiel 8

Gebleichter Baumwollfrottee wird auf einer Rouleaux-Druckmaschine mit 1 kg einer Druckpaste der Zusammensetzung

| | |
|---|---|
| 400 g | Alginatverdickungsmittel 5 %-ig, |
| 100 g | Harnstoff |
| 50 g | eines Farbstoffes der Formel |

(17) 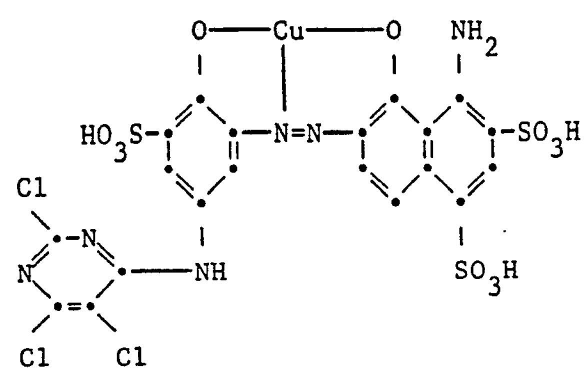

| | |
|---|---|
| 10 g | m-Nitrobenzolsulfonsäure (Natriumsalz) |
| 60 g | Natriumcarbonatlösung 30 %-ig und |

380 g Wasser

so bedruckt, dass jeweils 3 cm bedruckte Streifen neben 3 cm unbedruckten streifen resultieren. Die Ware wird dann getrocknet, 8 Minuten bei 101°C gedämpft, gespült, kochend geseift und getrocknet. Hierauf wird die bedruckte Ware mit einer Flotte, welche im Liter

23 g des gemäss Beispiel 1 hergestellten 1-(3'-Chlor-2'-hydroxypropylaminopropyl)-3-(3'-chlor-2'-hydroxypropyl)-imidazoliumsulfates und
30 ml Natriumhydroxydlösung (30 %)

enthält, foulardiert. Die Flottenaufnahme ist 85 %. Hiernach wird die Ware bei 80°C getrocknet und 3 Minuten bei 140°C behandelt. Anschliessend wir die Ware gespült und getrocknet. Die Nassechtheiten, insbesondere die ISO-C2S-Wäsche und die Nassbügelechtheit des Farbdruckes werden durch diese Nachbehandlung mit dem Imidazoliumsalz auch nach einem Hydrolysentest deutlich verbessert.
Ähnlich gute Effekte erhält man bei Verwendung von 23 g jedes der gemäss den Beispielen 2 bis 5 hergestellten Imidazoliumsalze.

## Patentansprüche

1. 1-Aminoalkylimidazoliumwerbindungen der Formel

(1)

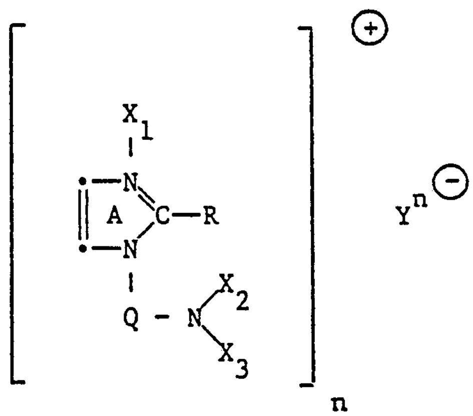

worin
$X^1$ und $X^2$, unabhängig voneinander die Gruppe

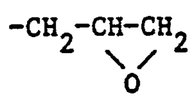

oder $-CH_2-CH-CH_2-Hal$,
|
OH

$X^3$ Wasserstoff, Niederalkyl,

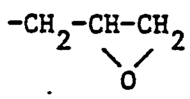

oder $-CH_2-CH-CH_2-Hal$,
|
OH

Hal ein Halogenatom,
Q Alkylen mit 2 bis 4 Kohlenstoffatomen,
R Wasserstoff, Phenyl oder einen aliphatischen Rest,
n 1 oder 2 und

$Y^{\ominus}$ ein Anion einer starken anorganischen oder organischen Säure bedeuten und der Imidazolring A unsubstituiert oder durch unsubstituiertes oder durch Halogen, Hydroxyl oder Cyano substituiertes Niederalkyl substituiert ist.

2. Imidazoliumverbindungen gemäss Anspruch 1, dadurch gekennzeichnet, dass in Formel (I) $X_1$ und $X_2$ die Halogenhydringruppen der Formel

$-CH_2-CH-CH_2-Hal_1$
        |
        OH

worin $Hal_1$ Brom oder Chlor ist, darstellen.

3. Imidazoliumverbindungen gemäss Anspruch 1 oder 2, dadurch gekennzeichnet, dass in Formel (I) $X_3$ Wasserstoff ist.

4. Imidazoliumverbindungen gemäss einem der Ansprüche 1 bis 3, dadurch gekennzeichnet, dass in Formel (I) Q die Propylengruppe $-CH_2-CH_2-CH_2-$ bedeutet.

5. Imidazoliumverbindungen gemäss Anspruch 1, dadurch gekennzeichnet, dass sie der Formel

(2)

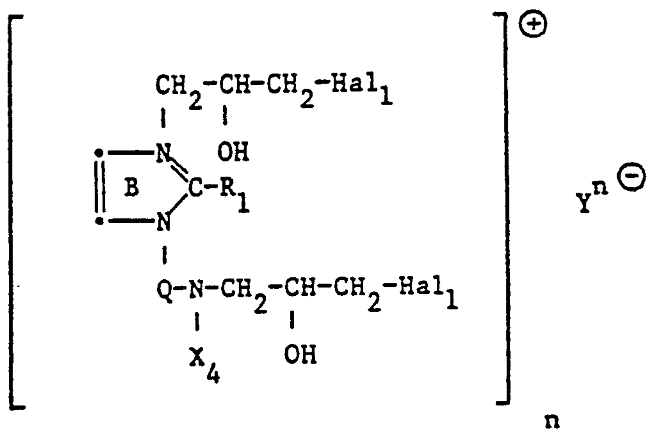

entsprechen, worin Q, $Y^{\ominus}$ und n die Anspruch 1 angegebene Bedeutung haben, $R_1$ Wasserstoff, Phenyl oder Alkyl mit 1 bis 17 Kohlenstoffatomen $X_4$ Wasserstoff, oder

$-CH_2-CH-CH_2-Hal_1$ und $Hal_1$ Brom oder
        |
        OH

Chlor bedeuten und der Ring B unsubstituiert oder Methyl oder Ethyl substituiert ist.

6. Imidazoliumverbindungen gemäss Anspruch 1, dadurch gekennzeichnet, dass sie der Formel

(3)

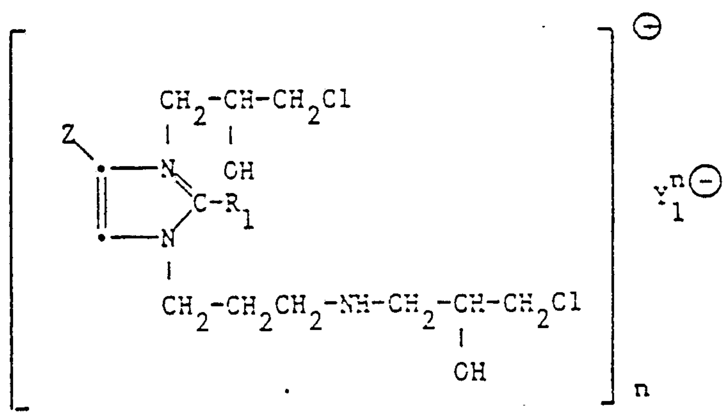

entsprechen worin $R^1$ Wasserstoff, Phenyl oder Alkyl mit 1 bis 17 Kohlenstoffatomen, Z Wasserstoff oder Methyl, n 1 oder 2 und $Y^n{}_1\ominus$ das Chlorid- oder Sulfation bedeuten.

7. Verfahren zur Herstellung von N-substituirten 1-Aminoalkyl-imidazoliumverbindungen dadurch gekennzeichnet, dass man 1 Mol eines 1-Aminoalkylimidazols oder dessen Säuresalzes mit 2 oder 3 Mol eines Epihalogenhydrins umsetzt.

8. Verfahren zur Verbesserung der Farbausbeute und der Nassechtheiten von mit anionischen Farbstoffen auf Cellulosefasermaterial erzeugten Färbungen oder Drucken, dadurch gekennzeichnet, dass man das

Fasermaterial vor, währen oder nach dem Färben mit Imidazoliumverbindungen gemäss Anspruch 1 behandelt.

9. Verfahren gemäss Anspruch 8, dadurch gekennzeichnet dass die Behandlung des Cellulosefasermaterials mit der Imidazoliumverbindung kontinuierlich gemäss einen Foulardierverfahren erfolgt.

10. Verfahren gemäss Anspruch 9, dadurch gekennzeichnet, dass die kontinuierliche Behandlung nach dem Färben erfolgt.

11. Verfahren gemäss einem der Ansprüche 8 bis 10, dadurch gekennzeichnet, dass die Behandlung des Cellulosematerials nach dem Färben durch Foulardieren des gefärbten oder bedruckten Fasermaterials mit anschliessendem Thermosolieren erfolgt.

12. Verfahren gemäss Anspruch 8, dadurch gekennzeichnet, dass die Behandlung des Cellulosefasermaterials mit der Imidazoliumverbindung gemäss dem Ausziehverfahren erfolgt.

13. Verfahren gemäss einem der Ansprüche 8 bis 12, dadurch gekennzeichnet, dass die Behandlung des Cellulosematerials mit der Imidazoliumverbindung aus alkalischem Medium erfolgt.

## Claims

1. A 1-aminoalkyl-imidazolium compound of the formula

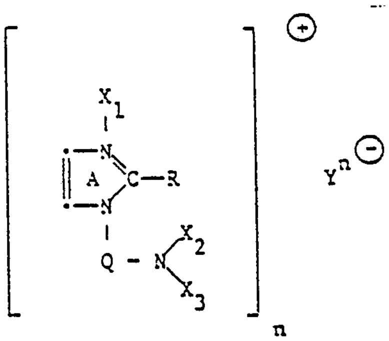

in which

$X_1$ and $X_2$ independently of one another are each the group

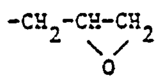

or $-CH_2-CH-CH_2-Hal$
|
OH

$X_3$ is hydrogen, lower alkyl,

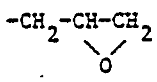

or
$-CH_2-CH-CH_2-Hal$
|
OH

Hal is a halogen atom,
Q is alkylene having 2 to 4 carbon atoms,
R is hydrogen, phenyl or an aliphatic radical,
n is 1 or 2, and
$Y^\ominus$ is an anion of a strong inorganic or organic acid, and the imidazole ring A is unsubstituted, or is substituted by lower alkyl which is unsubstituted or substituted by halogen, hydroxyl or cyano.

2. An imidazolium compound according to claim 1, wherein, in formula (1), $X_1$ and $X_2$ are the halohydrin groups of the formula

$-CH_2-CH-CH_2$ - $Hal_1$
|
OH

in which $Hal_1$ is bromine or chlorine.

3. An imidazolium compound according to claim 1 or 2, whereim, in formula (1), $X_3$ is hydrogen.

4. An imidazolium compound according to any one of claims 1 to 3, wherein, in formula (1), Q is the propylene group $-CH_2-CH_2-CH_2-$.

5. An imidazolium compound according to claim 1, which corresponds to the formula

(2) 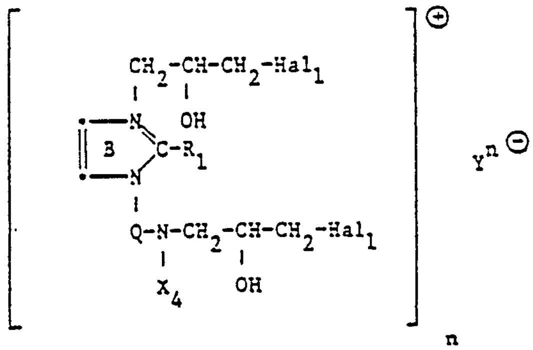

in which

Q, $Y^{\ominus}$ and n have the meaning defined in claim 1, $R_1$ is hydrogen, phenyl or alkyl having 1 to 17 carbon atoms, $X_4$ is hydrogen or
$-CH_2-CH-CH_2-Hal_1$, and $Hal_1$ is bromine or chlorine, and the ring B is
|
OH
unsubstituted or substituted by methyl or ethyl.

6. An imidazolium compound according to claim 1, which corresponds to the formula

(3) 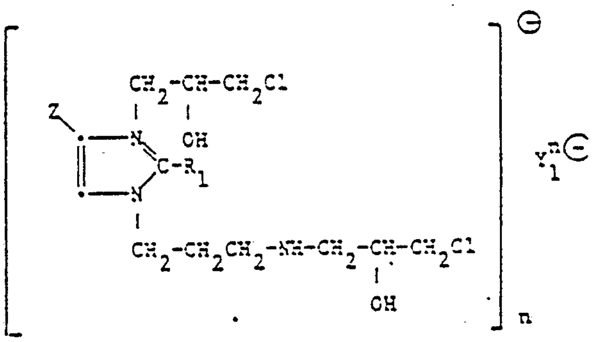

in which

$R_1$ is hydrogen, phenyl or alkyl having 1 to 17 carbon atoms, Z is hydrogen or methyl, n is 1 or 2, and $Y_1^{n\ominus}$ is the chloride or sulfate ion.

7. A process for the preparation of an N-substituted 1-aminoalkyl-imidazolium compound which comprises reacting 1 mole of a 1-aminoalkyl-imidazole or the acid salt thereof with 2 or 3 moles of an epihalohydrin.

8. A process for improving the dye yield and fastness properties of dyeings or print produced with anionic dyes on cellulose fibre material, which comprises treating the fibre material before, during or after dyeing with an imidazolium compound according to claim 1.

9. A process according to claim 8, wherein the treatment of the cellulose fibre material with the imidazolium compound is performed continuously by a padding process.

10. A process according to claim 9, wherein the continuous treatment is performed after dyeing.

11. A process according to any one of claims 8 to 10, wherein the treatment of the cellulose fibre material is performed, after dyeing, by padding of the dyed or printed fibre material with subsequent thermosol fixing.

12. A process according to claim 8, wherein the treatment of the cellulose fibre material with the imidazolium compound is performed by the exhaust process.

13. A process according to any one of claims 8 to 12, wherein the treatmemt of the cellulose material with the imidazolium compound is performed from an alkaline medium.

**Revendications**

1. Composés 1-aminoalkylimidazolium de formule

(1) 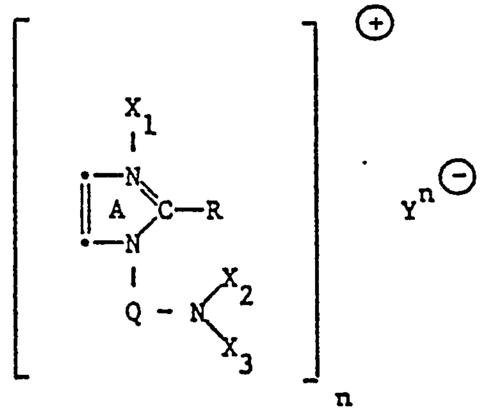

dans laquelle

$X_1$ et $X_2$ représentent, indépendamment l'un de l'autre, le groupe

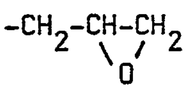

ou $-CH_2-CH-CH_2-Hal$;
|
OH

$X_3$ représente un atome d'hydrogène ou un groupe alkyle inférieur,

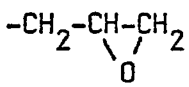

ou $-CH_2-CH-CH_2-Hal$;
|
OH

Hal représente un atome d'halogène;
Q représente un groupe alkylène ayant de 2 à 4 atomes de carbone;
R représente un atome d'hydrogène ou le radical phényle ou un radical aliphatique;
n vaut 1 ou 2; et
$Y^{\ominus}$ représente un anion d'un acide organique ou minéral fort; et le cycle imidazole A n'est pas substitué ou est substitué par un groupe alkyle inférieur non substitué ou substitué par un atome d'halogène ou par un radical hydroxyle ou cyano.

2. Composés imidazolium selon la revendication 1, caractérisés par le fait que dans la formule (1), $X_1$ et $X_2$ représentent les groupes halohydrine de formule

$-CH_2-CH-CH_2-Hal_1$
|
OH

dans laquelle $Hal_1$ représente le brome ou le chlore.

3. Composés imidazolium selon la revendication 1 ou 2, caractérisés par le fait que dans la formule (1), $X_3$ est un atome d'hydrogène.

4. Composés imidazolium selon l'une des revendications 1 à 3, caractérisés par le fait que dans la formule (1), Q représente le groupe propylène $-CH_2-CH_2-CH_2-$.

5. Composés imidazolium selon la revendication 1, caractérisés par le fait qu'ils correspondent à la formule

(2) 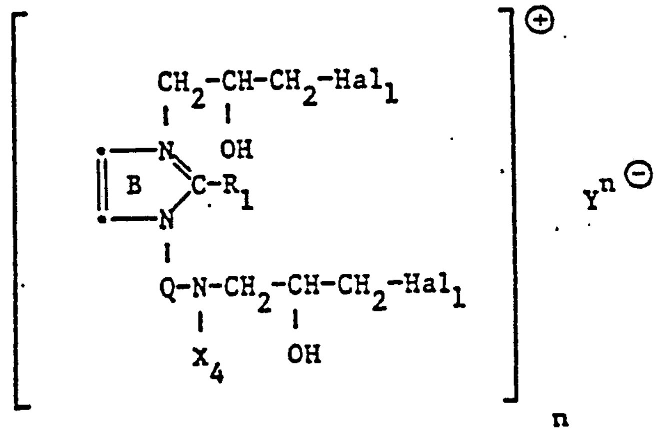

dans laquelle Q, $Y^{\ominus}$ et n ont les significations données dans la revendication 1, $R_1$ représente un atome d'hydrogène ou le groupe phényle ou un groupe alkyle ayant de 1 à 17 atomes de carbone, $X_4$ représente un atome d'hydrogène ou
$-CH_2-CH-CH_2-Hal_1$
|
OH
et $Hal_1$ représente le brome ou le chlore
et le cycle B n'est pas substitué ou est substitué par un groupe méthyle ou éthyle.

6. Composés imidazolium selon la revendication 1, caractérisés par le fait qu'ils correspondent à la formule

(3) 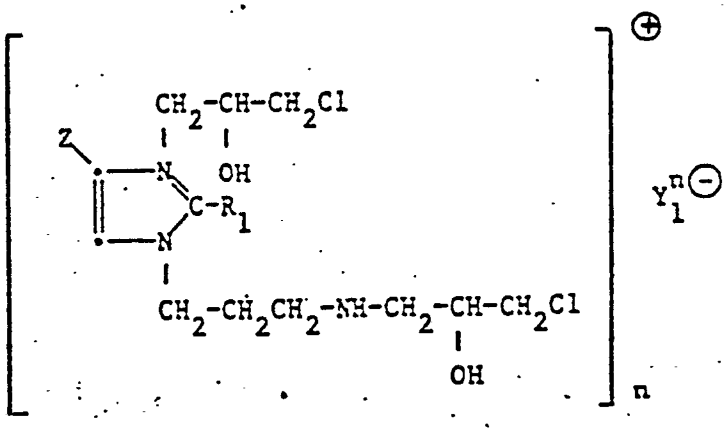

dans laquelle $R_1$ représente un atome d'hydrogène ou le groupe phényle ou un groupe alkyle ayant de 1 à 17 atomes de carbone, Z représente un atome d'hydrogène ou le groupe méthyle, n vaut 1 ou 2, et $Y_1^{n\ominus}$ représente l'ion chlorure ou sulfate.

7. Procédé pour la préparation de composés 1-aminoalkylimidazolium substitués à l'azote, caractérisé par le fait que l'on fait réagir 1 mole d'un 1-aminoalkylimidazole, ou d'un de ses sels avec un acide, avec 2 ou 3 moles d'une épihalohydrine.

8. Procédé pour l'amélioration du rendement tinctorial et de la solidité au mouillé de teintures ou impressions obtenues avec des colorants anioniques sur un matériau en fibres de cellulose, caractérisé par le fait que l'on traite le matériau fibreux, avant, pendant ou après la teinture, avec des composés imidazolium selon la revendication 1.

9. Procédé selon la revendication 8, caractérisé par le fait que l'on effectue le traitement du matériau en fibres de cellulose avec le composé imidazolium, en continu, selon un procédé de foulardage.

10. Procédé selon la revendication 9, caractérisé par le fait que l'on effectue le traitement en continu après la teinture.

11. Procédé selon l'une des revendications 8 à 10, caractérisé par le fait que l'on effectue le traitement du matériau cellulosique après la teinture, par foulardage du matériau fibreux teint ou imprimé, avec fixage subséquent par le procédé Thermosol.

12. Procédé selon la revendication 8, caractérisé par le fait que l'on effectue le traitement du matériau en fibres de cellulose avec le composé imidazolium, selon le procédé par épuisement.

13. Procédé selon l'une des revendications 8 à 12, caractérisé par le fait que l'on effectue le traitement du matériau cellulosique avec le composé imidazolium en milieu alcalin.